(19) Europäisches Patentamt
European Patent Office
Office européen des brevets

(11) Veröffentlichungsnummer: **0 065 480**
**B1**

(12) # EUROPÄISCHE PATENTSCHRIFT

(45) Veröffentlichungstag der Patentschrift: **14.11.84**

(51) Int. Cl.³: **C 07 D 239/30**, C 09 B 62/20

(21) Anmeldenummer: **82710026.4**

(22) Anmeldetag: **03.05.82**

(54) **Im Pyrimidinkern monofluorierte seitenkettenhaltige Halogenpyrimidine, Verfahren zu ihrer Herstellung und ihre Verwendung zur Herstellung von Reaktivfarbstoffen.**

(30) Priorität: **12.05.81 DE 3118700**

(43) Veröffentlichungstag der Anmeldung: **24.11.82 Patentblatt 82/47**

(45) Bekanntmachung des Hinweises auf die Patenterteilung: **14.11.84 Patentblatt 84/46**

(84) Benannte Vertragsstaaten: **CH DE FR GB IT LI**

(56) Entgegenhaltungen:
**EP - A - 0 004 945**
**DE - A - 1 670 780**
**GB - A - 1 157 948**
**US - A - 3 314 955**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Klauke, Erich, Dr., Eichendorffweg 8, D-5068 Odenthal (DE)**
Erfinder: **Schallner, Otto, Dr., Jakob-Böhme-Strasse 11, D-5000 Köln 80 (DE)**
Erfinder: **Schündehütte, Karl Heinz, Prof. Dr., Klief 75, D-5090 Leverkusen 3 (DE)**

EP 0 065 480 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue im Pyrimidinkern monofluorierte seitenkettenhaltige Halogenpyrimidine der allgemeinen Formel I

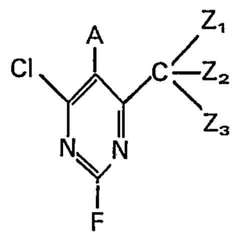

(I)

wobei A=H, Cl oder Br, $Z_1$=H oder Cl, $Z_2$=H oder Cl, und $Z_3$=Cl.

Insbesondere bevorzugt ist das Halogenpyrimidin der Formel II

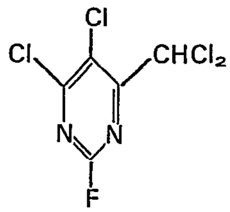

(II)

Die Erfindung betrifft weiterhin ein Verfahren zur Herstellung von Halogenpyrimidinen der allgemeinen Formel III

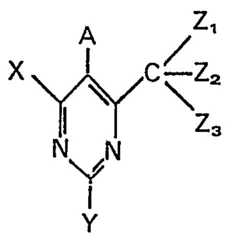

(III)

worin X ≠ Y und jeweils für Chlor oder Fluor steht, $Z_1$=H oder Cl, $Z_2$=H oder Cl, $Z_3$=Cl, und A=H, Cl oder Br.

Das Verfahren ist dadurch gekennzeichnet, dass man

a) zum Erhalt von Verbindungen der allgemeinen Formel III, in welcher X für F und Y für Cl steht und A, $Z_1$, $Z_2$ und $Z_3$ die oben angegebene Bedeutung besitzen, Pyrimidine der allgemeinen Formel IV

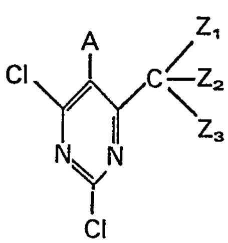

(IV)

mit etwa der äquimolaren Menge Natriumfluorid in Tetramethylensulfon bei Temperaturen von ca. 80 bis ca. 180° C umsetzt,

und dass man

b) zum Erhalt von Verbindungen der allgemeinen Formel III, in welcher X für Cl und Y für F steht, in einem Autoklaven pro ca. 1 bis ca. 40 mol wasserfreier Flusssäure ca. 1 mol der Verbindung der allgemeinen Formel IV bei Temperaturen von ca. 40 bis ca. 160° C umsetzt, wobei der entstehende Chlorwasserstoff über ein Ventil so entspannt wird, dass der Innendruck konstant bleibt.

Das Reaktionsschema sei im folgenden skizziert

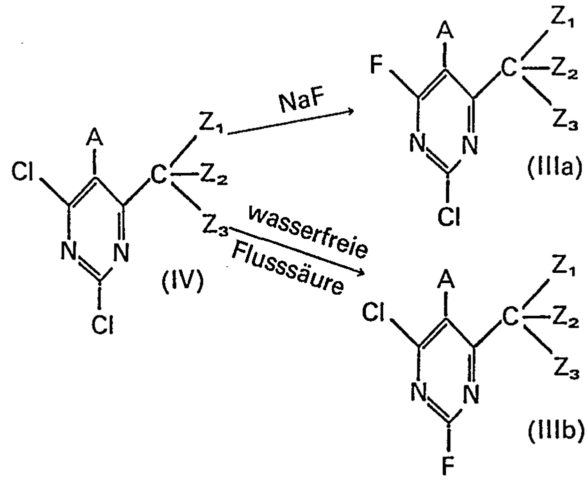


(IIIa) (IV) (IIIb)

wobei A, $Z_1$, $Z_2$ und $Z_3$ die oben angegebene Bedeutung besitzen.

Die für die Herstellung der Verbindungen der allgemeinen Formel III einzusetzenden Chlorpyrimidinderivate der allgemeinen Formel IV sind zum Teil bekannt. Sie werden nach an sich bekannten Methoden hergestellt (Literatur: s. z.B. „Journal of Het. Chem.", *16*, 1575 [1979]). Folgende Verbindungen der allgemeinen Formel IV sind als Ausgangsverbindungen für III von besonderem Interesse

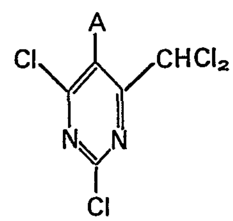

(IVa)

wobei A, H, Cl oder Br bedeutet.

Die Verbindungen der allgemeinen Formel IVa werden hergestellt, indem man die entsprechenden, in 5-Stellung des Pyrimidinmoleküls H, Cl oder Br enthaltenden 6-Methyluracile zunächst mit $POCl_3$ in die 2,4-Dichlor-5-(H,Cl,Br)-6-methyluracile überführt und diese anschliessend ohne Zwischenisolierung mit elementarem Chlor bei Temperaturen von ca. 60 bis ca. 160° C behandelt. Von besonderem Wert als Ausgangsverbindung der allgemeinen Formel IV ist

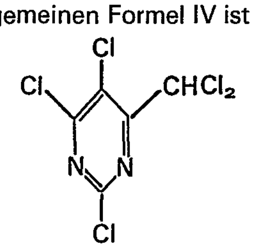

Im Pyrimidinkern sind Halogenatome in 2,4- und 6-Stellung durch den Einfluss der Ringstickstoffatome aktiviert. Ihr Austausch gegen Fluor erfolgt im allgemeinen durch Kaliumfluorid in polaren Lösungsmitteln wie z.B. Tetramethylensulfon,

Dimethylsulfoxid, N-Methylpyrrolidon oder anderen (vgl. z.B. M. Hudlicky, „Chemistry of Organic Fluorine Compounds", 2. Aufl., J. Wiley and Sons *1976*, S. 123-124). In einigen Fällen ist auch die Verwendung von Natriumfluorid beschrieben worden (s.o. S. 129). B. A. Ivin *et al.* beschreiben in „J. Gen. Chem. USSR", vol. 34, S. 4283 (engl. Übers.) die Herstellung von 2,4-Difluor-6-methylpyrimidin und von 2,4-Difluor-5-brommethylpyrimidin durch einen Austausch des Chlors gegen ein Fluoratom mit Kaliumfluorid. Wegen extremer Schwierigkeiten schlagen die Autoren Massnahmen vor, wie Einsatz bestimmter Katalysatoren und Lösungsmittel die Ausbeute auf schliesslich 40% der Theorie bringen.

Weiterhin ist bekannt, dass man Trichlormethyl- und auch Dichlormethylgruppen in der Seitenkette von aromatischen Verbindungen durch Cl/F-Austausch herstellen kann (s. u.a. Houben-Weyl, Bd. 5/3, S. 122; M. Hudlicky, s.o. S. 96, 105).

Wie oben beschrieben, wurde nun gefunden, dass man die neuen in 2- und 4-Stellung fluorierten Pyrimidine der allgemeinen Formel I durch einen Austausch von Chlor gegen Fluoratome erhalten kann. Als Fluorierungsmittel kann man, wie oben erwähnt, sowohl wasserfreie Fluorwasserstoffsäure als auch Alkalifluorid (insbesondere Natriumfluorid) im Lösungsmittel verwenden. Das ist überraschend, denn durch die bisher bekannt gewordenen Fluorierungen mit den vorgenannten Fluorierungsmitteln ist eine selektive Chlor/Fluor-Austauschreaktion am Kern des Pyrimidinringes weder beschrieben noch nahegelegt worden.

Der selektive Austausch von Chlor gegen Fluor in 4-Stellung des Pyrimidinkerns mit Natriumfluorid wird bevorzugt bei Temperaturen von ca. 100 bis ca. 180° C durchgeführt. Pro Mol Ausgangsmaterial der allgemeinen Formel IV werden 0,8 bis 1,2 mol Natriumfluorid eingesetzt. Die Selektivität der Umsetzung ist praktisch quantitativ. Im allgemeinen werden mehr als 95% der Verbindung der allgemeinen Formel IIIa erhalten.

Der Austausch des Chloratoms in 2-Stellung des Pyrimidinrings mit wasserfreier Flusssäure gemäss Verfahrensvariante b erfolgt ebenfalls mit hoher Selektivität von über 90% und ist in seinem Verlauf überraschend und keineswegs vorhersehbar, denn es ist bisher noch nicht bekannt geworden, dass die Chlor/Fluor-Austauschreaktion an Heterocyclen mit HF nach einem anderen Substitutionsmuster verläuft als diejenige mit Alkalifluoriden. Die Umsetzung von Verbindungen der allgemeinen Formel IV mit wasserfreier Flusssäure wird so durchgeführt, dass man die wasserfreie Flusssäure bei Temperaturen unter 20° C in einem Autoklaven vorlegt, das Ausgangsmaterial zutropft und dann das Reaktionsgefäss verschliesst. Der Ansatz wird schliesslich auf Reaktionstemperatur aufgeheizt und das entstehende Chlorwasserstoffgas über ein Ventil so entspannt, dass der Innendruck im Autoklaven konstant bleibt. Bei dieser letztgenannten Umsetzung setzt man pro Mol der Ausgangskomponente der allgemeinen Formel IV 1 bis 40 mol Fluorwasserstoff hinzu, vorzugsweise pro Mol Ausgangskomponente IV 10 bis 20 mol Fluorwasserstoff, d.h. die wasserfreie Flusssäure dient bei dieser Umsetzung gleichzeitig als Lösungsmittel für die Reaktion. Diese Reaktion wird in einem Temperaturbereich von ca. 40 bis ca. 160, vorzugsweise zwischen ca. 60 bis ca. 100° C durchgeführt. Die Aufarbeitung des Reaktionsproduktes der allgemeinen Formel I geschieht im allgemeinen durch Destillation. Man kann aber auch zunächst durch Zusatz von Wasser aufarbeiten und anschliessend die organische Phase getrennt abdestillieren.

Die Verbindungen der allgemeinen Formel I sind wertvolle Zwischenprodukte für die Herstellung von Reaktivfarbstoffen mit Halogenpyrimidinylreaktivresten. Die Reaktivfarbstoffe werden erhalten durch Umsetzung von aminogruppen- bzw. carbonamidgruppenhaltigen chromophoren Systemen mit den Verbindungen der allgemeinen Formel I unter Zusatz von Alkali zur Bindung des freiwerdenden Halogenwasserstoffs. Die aus den Verbindungen der allgemeinen Formel I durch Umsetzung mit chromophoren Systemen hergestellten neuen Reaktivfarbstoffe zeigen ein im überraschenden Masse besseres Ziehvermögen im technisch relevanten Klotz-Kaltverweil-Verfahren als die nächstverwandten Vergleichsfarbstoffe aus DE-A Nr. 2817780.

*Beispiel 1*

In einer Druckapparatur aus rostfreiem Stahl mit Rührer und Rückflusskühler mit nachgeschaltetem Entspannungsventil werden bei etwa 0° C 160 ml wasserfreie Flusssäure vorgelegt. Hierzu lässt man 301 g (1 mol) 2,4,5-Trichlor-6-trichlormethylpyrimidin zutropfen, verschliesst die Apparatur und erzeugt einen Überdruck von 3 bar Stickstoff. Dann wird auf eine Temperatur von 80° C hochgeheizt. Durch die bei der Reaktion im Autoklaven freiwerdende Chlorwasserstoffsäure steigt der Druck im Autoklaven an. Man hält diesen Druck über ein Entspannungsventil bei 15 bar konstant. Nach 5 h Reaktionszeit wird der Ansatz abgekühlt, entspannt und durch Destillation aufgearbeitet. Nach einem geringen Vorlauf erhält man 103 g 2-Fluor-4,5-dichlor-6-trichlormethylpyrimidin vom Siedepunkt 129 bis 130° C bei 10 mbar Druck.

$n_D^{20}$: 1,5618.

$^{19}$F-NMR ist die erhaltene Substanz 96%ig und enthält nur 4% der isomeren Verbindung 2,5-Dichlor-4-fluor-6-trichlormethylpyrimidin.

*Beispiel 2*

In einem 500-ml-Rührgefäss mit Rührer, Thermometer, Rückflusskühler und einer elektrischen Heizung werden 150 g 2,4,5-Trichlor-6-trichlormethylpyrimidin, 31 g Natriumfluorid (entsprechend einem Molverhältnis von 1:1,5) und 90 ml Tetramethylensulfon vorgelegt. Man erhöht die Temperatur auf 160° C und rührt ca. 3 h bei dieser Temperatur. Dann lässt man auf ca. 80° C abkühlen und filtriert ab. Das Filtrat wird fraktioniert destilliert. Nach einem geringen Anteil 2,4-Difluor-5-chlor-6-trichlormethylpyrimidin im Vorlauf isoliert

man 85 g 4-Fluor-2,5-dichlor-6-trichlormethyl-pyrimidin vom Siedepunkt 124° C bei 15 mbar.

$n_D^{20}$: 1,5545.

Die Substanz ist laut $^{19}F$-NMR-Spektroskopie 100%ig rein und ist frei von der isomeren 2-Fluorverbindung.

*Beispiel 3*

In einem Dreihals-Rührkolben aus Glas werden 180 g 2,4,5-Trichlor-6-dichlormethylpyrimidin und 85 g Natriumfluorid in 240 ml Tetramethylensulfon (wasserfrei) vorgelegt. Man heizt den Ansatz unter Rühren auf 120° C auf und erhöht die Temperatur nach 2 h auf 140° C. Nach weiteren 2 h kühlt man ab und versetzt den Ansatz mit Wasser. Die organische Phase wird abgetrennt, die wässerige Phase einmal mit Methylenchlorid extrahiert. Die organischen Anteile werden vereinigt, nochmals mit Wasser ausgewaschen und dann getrocknet. Nach Abdestillieren des Lösungsmittels erhält man 155 g einer Flüssigkeit.

$n_D^{20}$: 1,5440.

Durch fraktionierte Destillation erhält man hieraus nach einem kleinen Vorlauf 64 g 4-Fluor-2,5-dichlor-6-dichlormethylpyrimidin vom Siedepunkt 117° C/12 mbar.

$n_D^{20}$: 1,5553.

Nach dem $^{19}F$-NMR-Spektrum ist die Verbindung frei von Isomeren. Die höhersiedenden Nachläufe können in einer weiteren Fluorierung wieder eingesetzt werden.

*Beispiel 3a*

In einen 4-l-Vierhalskolben mit Rührer, Rückflusskühler, Thermometer und Gaseinleitungsrohr werden 1975 g (≙ 10 mol) 6-Methyl-2,4,5-trichlorpyrimidin und 200 ml Phosphoroxychlorid vorgelegt. Man erwärmt die Mischung auf 90° C und leitet über eine Zeit von ca. 8 h und unter kräftigem Rühren 1200 g Chlorgas ein. Danach wird das Reaktionsgemisch unter teilweisem Abdestillieren des Phosphoroxychlorids auf 120° C erwärmt. Es werden über 4 h weitere 350 g Chlorgas in das Reaktionsgemisch eingeleitet. Danach ist die Reaktion beendet. Der Endpunkt kann gaschromatographisch ermittelt werden. Man destilliert das restliche Phosphoroxychlorid ab und unterwirft den Rückstand einer Vakuumdestillation.

Ausbeute: 2040 g 6-Dichlormethyl-2,4,5-trichlorpyrimidin ($Kp._{0,2\,mbar}$=92° C).

$^{1}H$=NMR-Spektrum ($CDCl_3$):δ=(s, 1H).

Verwendet man anstelle von 6-Methyl-2,4,5-trichlorpyrimidin 2304 g 5-Brom-2,4-dichlor-6-methylpyrimidin und verfährt sonst wie oben beschrieben, so erhält man 2370 g 5-Brom-6-dichlormethyl-2,4-dichlorpyrimidin ($Kp._{0,01\,mbar}$ =82° C).

*Beispiel 4*

In einer Apparatur wie in Beispiel 1 beschrieben, legt man bei etwa 0° C 400 ml wasserfreie Flusssäure vor, gibt 266,5 g 2,4,5-Trichlor-6-dichlormethylpyrimidin zu und verschliesst dann den Autoklaven. Unter Rühren wird bis auf 60° C geheizt. Bei dieser Temperatur lässt man den Ansatz ausreagieren. Durch die freiwerdende Chlorwasserstoffsäure steigt der Innendruck an. Man hält den Innendruck durch Entspannen über das Ventil bei etwa 5 bar konstant. Danach wird abgekühlt, der Autoklav vollständig entspannt und die überschüssige HF abdestilliert. Der Rückstand wird auf Eis gegeben, die organische Phase abgetrennt und der wässerige Anteil einmal mit Methylenchlorid extrahiert. Die vereinigten organischen Phasen werden über Natriumsulfat getrocknet und durch Destillation aufgearbeitet. Nach einem Vorlauf von etwas 2,4-Difluor-5-chlor-5-dichlormethylpyrimidin erhält man 74 g 2-Fluor-4,5-dichlor-6-dichlormethylpyrimidin vom Siedepunkt 120° C bei einem Druck von 12 mbar. Reinheit: 91%.

$n_D^{20}$: 1,5562.

Aus dem langsam kristallisierenden Öl entsteht ein Festprodukt, welches nach einmaligem Umkristallisieren aus Petrolether einen Schmelzpunkt von 45° C aufweist.

*Beispiel 5*

Auf die gleiche Weise wie in Beispiel 4 beschrieben, erhält man aus 2,4-Dichlor-5-brom-6-dichlormethylpyrimidin die Monofluorverbindung 2-Fluor-4-chlor-5-brom-6-dichlormethylpyrimidin vom Siedepunkt 120° C bei einem Druck von 12 mbar. Der Schmelzpunkt beträgt 55 bis 56° C.

*Beispiel 6*

Auf die gleiche Weise wie in Beispiel 4 beschrieben, erhält man aus 2,4-Dichlor-6-trichlormethylpyrimidin die Monofluorverbindung 2-Fluor-4-chlor-6-trichlormethylpyrimidin. $Kp._{16\,mbar}$=110-111° C.

$n_D^{20}$: 1,540, langsam kristallisierend.

F. 30-31° C.

*Beispiel 7*

Auf die gleiche Weise wie in Beispiel 2 beschrieben, erhält man aus 2,4-Dichlor-5-brom-6-dichlormethylpyrimidin die Monofluorverbindung 2-Chlor-4-fluor-5-brom-6-dichlormethylpyrimidin.

*Beispiel 8*

Auf die gleiche Weise wie in Beispiel 2 beschrieben, erhält man aus 2,4-Dichlor-6-trichlormethylpyrimidin die Monofluorverbindung 2-Chlor-4-fluor-6-trichlormethylpyrimidin. $Kp._{12\,mbar}$ =112°C. F. 67° C.

## Patentansprüche

1. Halogenpyrimidine der allgemeinen Formel (I)

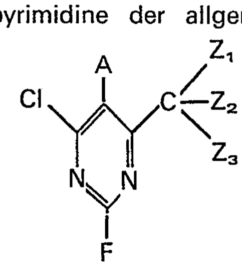

(I)

wobei A=H, Cl oder Br, $Z_1$=H oder Cl, $Z_2$=H oder Cl, und $Z_3$=Cl.

2. Halogenpyrimidin der Formel (II)

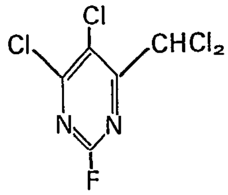

(II)

3. Verfahren zur Herstellung von Halogenpyrimidinen der allgemeinen Formel (III)

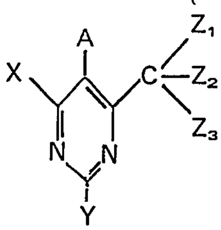

(III)

worin X ≠ Y und jeweils für Chlor oder Fluor steht, $Z_1$=H oder Cl, $Z_2$=H oder Cl, $Z_3$=Cl, und A=H, Cl oder Br bedeuten,
dadurch gekennzeichnet, dass man

a) zum Erhalt von Verbindungen der allgemeinen Formel (III), in welcher X für F und Y für Cl steht und A, $Z_1$, $Z_2$ und $Z_3$ die oben angegebene Bedeutung besitzen, Pyrimidine der allgemeinen Formel (IV)

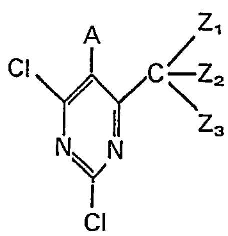

(IV)

mit etwa der äquimolaren Menge Natriumfluorid in Tetramethylensulfon bei Temperaturen von ca. 80 bis ca. 180° C umsetzt,
und dass man

b) zum Erhalt von Verbindungen der allgemeinen Formel (III), in welcher X für Cl und Y für F steht, in einem Autoklaven pro ca. 1 bis ca. 40 mol wasserfreier Flusssäure ca. 1 mol der Verbindung der allgemeinen Formel (IV) bei Temperaturen von ca. 40 bis ca. 160° C umsetzt, wobei der entstehende Chlorwasserstoff über ein Ventil so entspannt wird, dass der Innendruck konstant bleibt.

4. Verwendung der Halogenpyrimidine der allgemeinen Formel (III) zur Herstellung von Reaktivfarbstoffen.

**Claims**

1. Halogenopyrimidines of the general Formula (I)

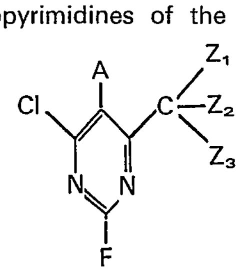

(I)

wherein A=H, Cl or Br, $Z_1$=H or Cl, $Z_2$=H or Cl, and $Z_3$=Cl.

2. Halogenopyrimidine of the Formula (II)

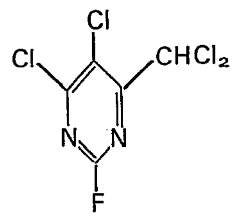

(II)

3. Process for the preparation of halogenopyrimidines of the general Formula (III)

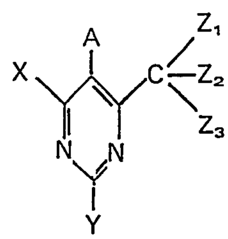

(III)

wherein X ≠ Y and each represents chlorine or fluorine, $Z_1$ denotes H or Cl, $Z_2$ denotes H or Cl, $Z_3$ denotes Cl and A denotes H, Cl or Br,
characterised in that

a) to obtain compounds of the general Formula (III), in which X represents F and X represents Cl and A, $Z_1$, $Z_2$ and $Z_3$ have the meaning indicated above, pyrimidines of the general Formula (IV)

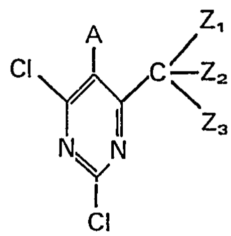

(IV)

are reacted with an approximately equimolar quantity of sodium fluoride in tetramethylene sulphone at temperatures of about 80 to about 180° C,
and in that

b) to obtain compounds of the general Formula (III), in which X represents Cl and Y represents F, about 1 mol of the compound of the general Formula (IV) to about 1 to about 40 mol of anhydrous hydrofluoric acid are reacted in an autoclave at temperatures of about 40 to about 160° C, the hydrogen chloride which forms being released *via* a valve in such a manner that the internal pressure remains constant.

4. Use of the halogenopyrimidines of the general Formula (III) for the preparation of reactive dyestuffs.

**Revendications**

1. Halogénopyrimidines de formule générale (I)

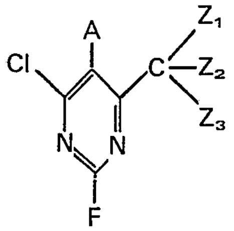

(I)

dans laquelle A=H, Cl ou Br, $Z_1$=H ou Cl, $Z_2$=H ou Cl, et $Z_3$=Cl.

2. Halogénopyrimidine de formule (II)

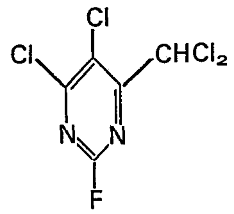

(II)

3. Procédé de préparation des halogénopyrimidines de formule générale (III)

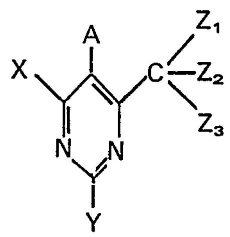

(III)

dans laquelle X et Y sont différents et représentent chacun le chlore ou le fluor, $Z_1$=H ou Cl, $Z_2$=H ou Cl, $Z_3$=Cl, A=H, Cl ou Br,
caractérisé en ce que

a) pour obtenir les composés de formule générale (III) dans laquelle X représente F et Y représente Cl, A, $Z_1$, $Z_2$ et $Z_3$ ayant les significations indiquées ci-dessus, on fait réagir des pyrimidines de formule générale (IV)

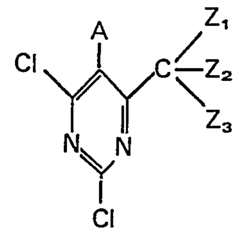

(IV)

avec la quantité à peu près équimoléculaire de fluorure de sodium dans la tétraméthylènesulfone à des températures d'environ 80 à 180° C, et

b) pour obtenir les composés de formule générale (III) dans laquelle X représente Cl et Y représente F, on fait réagir dans un autoclave, pour environ 1 à 40 mol d'acide fluorhydrique anhydre, environ 1 mol du composé de formule générale (IV) à des températures d'environ 40 à 160° C, en détendant le chlorure d'hydrogène formé au travers d'une soupape de manière que la pression intérieure reste constante.

4. Utilisation des halogénopyrimidines de formule générale (III) pour la préparation de colorants réactifs.